(19) Europäisches Patentamt
European Patent Office
Office européen des brevets

(11) Veröffentlichungsnummer : **0 055 208**
**B1**

(12) **EUROPÄISCHE PATENTSCHRIFT**

(45) Veröffentlichungstag der Patentschrift : **13.03.85**

(21) Anmeldenummer : **81730124.5**

(22) Anmeldetag : **17.12.81**

(51) Int. Cl.⁴ : **C 07 C 59/62**, C 07 C 69/66, C 07 C103/175, C 07 C103/90, C 07 C143/74, A 61 K 31/00// C07C177/00, A61K31/557

(54) **Neue Carbacycline, Verfahren zu ihrer Herstellung und ihre Verwendung als Arzneimittel.**

(30) Priorität : **19.12.80 DE 3048906**

(43) Veröffentlichungstag der Anmeldung : **30.06.82 Patentblatt 82/26**

(45) Bekanntmachung des Hinweises auf die Patenterteilung : **13.03.85 Patentblatt 85/11**

(84) Benannte Vertragsstaaten : **AT BE CH DE FR GB IT LI LU NL SE**

(56) Entgegenhaltungen : **EP-A- 0 036 730**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Skuballa, Werner, Dr.**
**Olwenstrasse 25**
**D-1000 Berlin 28 (DE)**
Erfinder : **Radüchel, Bernd, Dr.**
**Gollanczstrasse 132**
**D-1000 Berlin 28 (DE)**
Erfinder : **Schwarz, Norbert, Dr.**
**Hauptstrasse 118**
**D-1000 Berlin 62 (DE)**
Erfinder : **Vorbrüggen, Helmut, Prof. Dr.**
**Wilkestrasse 7**
**D-1000 Berlin 27 (DE)**
Erfinder : **Casals-Stenzel, Jorge, Dr.**
**Wichernstrasse 20**
**D-1000 Berlin 33 (DE)**
Erfinder : **Schillinger, Ekkehard, Dr.**
**Im Amseltal 50**
**D-1000 Berlin 28 (DE)**
Erfinder : **Town, Michael Harold, Dr.**
**Buchsbaumweg 3**
**D-1000 Berlin 47 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 055 208 B1

Jouve, 18, rue St-Denis, 75001 Paris, France

**Beschreibung**

Die Erfindung betrifft neue Prostacyclin-Derivate, Verfahren zu ihrer Herstellung sowie ihre Verwendung als Arzneimittel.

In den deutschen Offenlegungsschriften DE-OS 28 45 770, 29 00 352, 29 02 442, 29 04 655, 29 09 088 und 29 12 409 werden (5E)- und (5Z)-6a-Carbaprostaglandin-$I_2$-Analoga beschrieben. Die Nomenklatur der erfindungsgemäßen Verbindungen basiert auf einem Vorschlag von Morton und Brokaw (J. Org. Chem. *44*, 2880 [1979]. Bei der Synthese dieser Verbindungen entstehen stets zwei Doppelbindungsisomere, die durch den Zusatz (5E) oder (5Z) charakterisiert werden. Die beiden Isomeren dieses Prototyps werden durch folgende Strukturformel verdeutlicht :

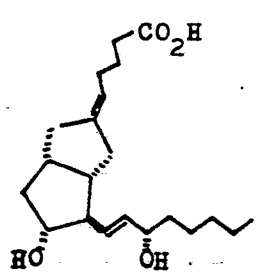

(5E)-6a-Carbaprostaglandin-$I_2$

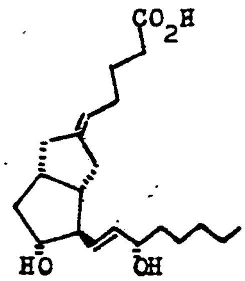

(5Z)-6a-Carbaprostaglandin-$I_2$

Aus dem sehr umfangreichen Stand der Technik der Prostacycline und ihrer Analoga weiß man, daß diese Stoffklasse aufgrund ihrer biologischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

Es wurde nun gefunden, daß durch Substitution der Methylengruppe in der 3-Position des Carbacyclins durch Sauerstoff eine längere Wirkungsdauer, größere Selektivität und bessere Wirksamkeit erzielt werden können. Die erfindungsgemäßen Verbindungen wirken bronchodilatatorisch und sind zur Inhibierung der Thrombozytenaggregation, zur Blutdrucksenkung über eine Vasodilation und zur Hemmung der Magensäuresekretion geeignet.

Die Erfindung betrifft Carbacyclinderivate der allgemeinen Formel I

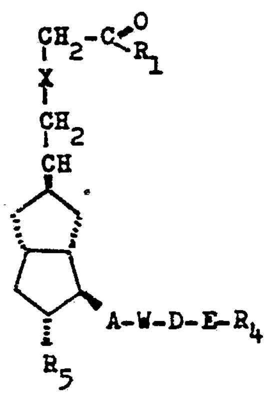

(I)

worin,

$R_1$ den Rest $OR_2$, wobei $R_2$ Wasserstoff, einen Alkylrest mit 1-10 C-Atomen, der durch Fluor, Chlor, Brom, Phenyl, Dimethylamino, Diethylamino, Methoxy oder Ethoxy substituiert sein kann, einen Cycloalkylrest mit 5-6 C-Atomen, der durch einen Alkylrest mit 1-4 C-Atomen substituiert sein kann, einen Phenylrest oder die Reste 2- oder 3-Furyl, 2- oder 3-Thienyl oder 2-, 3- oder 4-Pyridyl bedeuten kann, oder den Rest $NHR_3$ mit $R_3$ in der Bedeutung einer Carbonsäure oder Sulfonsäure mit 1-15 C-Atomen oder eines Wasserstoffatoms,

X Sauerstoff

A eine $-CH_2-CH_2-$, trans-CH=CH- oder $-C{\equiv}C$-Gruppe ;

W eine freie oder funktionell abgewandelte Hydroxymethylengruppe oder eine freie oder funktionell abgewandelte

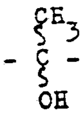

Gruppe, wobei die OH-Gruppe α- oder β-ständig sein kann,

D eine geradkettige oder verzweigte, gesättigte oder ungesättigte Alkylengruppe mit 1-10 C-Atomen, die durch Fluoratome, 1,2-Methylen oder 1,1-Trimethylen substituiert sein kann,

E eine —C≡C-Gruppe oder eine —$CR_6$=$CR_7$-Gruppe darstellt, wobei $R_6$ und $R_7$ ein Wasserstoffatom oder eine Alkylgruppe mit 1-5 C-Atomen bedeuten,

$R_4$ eine Alkylgruppe mit 1-10 C-Atomen, eine Cycloalkylgruppe mit 5-6 C-Atomen, Phenyl oder die Reste 2- oder 3-Furyl, 2- oder 3-Thienyl- oder 2-, 3- oder 4-Pyridyl,

$R_5$ eine freie oder funktionell abgewandelte Hydroxygruppe,

und, falls $R_2$ die Bedeutung eines Wasserstoffatoms hat, deren Salze mit physiologisch verträglichen Basen bedeuten.

Die Verbindungen der Formel I stellen sowohl (5E)- als auch (5Z)-Isomere dar.

Als Alkylgruppen $R_2$ sind gerad- oder verzweigtkettige Alkylgruppen mit 1-10 C-Atomen zu betrachten, wie beispielsweise Methyl, Äthyl, Propyl, Isopropyl, Butyl, Isobutyl, tert.-Butyl, Pentyl, Isopentyl, Neopentyl, Heptyl, Hexyl, Decyl.

Als bevorzugte Alkylgruppen $R_2$ sind solche mit 1-4 C-Atomen, wie z. B. Methyl, Äthyl, Propyl, Dimethylaminopropyl, Isobutyl, Butyl zu nennen.

Die Cycloalkylgruppe $R_2$ enthält im Ring 5 oder 6 Kohlenstoffatome. Die Ringe können durch Alkylgruppen mit 1-4 Kohlenstoffatomen substituiert sein. Beispielsweise seien genannt Cyclopentyl-, Cyclohexyl, Methylcyclohexyl.

Als Säurerest $R_3$ kommen physiologisch verträgliche Säurereste organischer Carbonsäuren und Sulfonsäuren mit 1-15 Kohlenstoffatomen, die der aliphatischen, cycloaliphatischen, aromatischen, aromatisch-aliphatischen und heterocyclischen Reihe angehören, in Frage. Diese Säuren können gesättigt, ungesättigt, mehrbasisch und in üblicher Weise substituiert sein. Als Beispiele für die Substituenten seien $C_1$-$C_4$-Alkyl-, Hydroxy-, $C_1$-$C_4$-Alkoxy-, Oxo- oder Aminogruppen oder Halogenatome (F, Cl, Br) erwähnt.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Methansulfonsäure, Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis-(β-chloräthyl)-aminosulfonsäure, N,N-Diisobutylaminosulfonsäure, N,N-Dibutylaminosulfonsäure, Pyrolidino-, Piperidino-, Piperazino-, N-Methylpiperazino- und Morpholinosulfonsäure in Frage.

Die Hydroxygruppen $R_5$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind.

Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butyl-silyl- und Tri-p-benzyl-silylrest. Als Acylreste kommen die gleichen wie für $R_3$ genannt in Frage; namentlich genannt seien beispielsweise Acetyl, Propionyl, Butyryl, Benzoyl.

Als Alkylgruppe $R_4$ kommen gerad- und verzweigtkettige, gesättigte und ungesättigte Alkylreste, vorzugsweise gesättigte, mit 1-10, insbesondere 1-7 C-Atomen, in Frage.

Beispielsweise genannt seien Methyl-, Äthyl-, Propyl-, Butyl-, Isobutyl-, tert.-Butyl-, Pentyl-, Hexyl-, Heptyl-, Octyl-, Butenyl-, Isobutenyl-, Propenyl-, Pentenyl- und Hexenyl-.

Die Cycloalkylgruppe $R_4$ enthält im Ring 5 oder 6 Kohlenstoffatome.

Als Alkylengruppe D kommen geradkettige oder verzweigtkettige, gesättigte und ungesättigte Alkylenreste mit 1-10 C-Atomen, vorzugsweise gesättigte, insbesondere mit 1-5 C-Atomen, in Frage, die durch Fluoratome, 1,2-Methylen oder 1,1-Trimethylen substituiert sein können. Beispielsweise seien genannt: Methylen, Fluormethylen, Pentamethylen, 1-Methyltetramethylen, 1-Methyl-trimethylen, 1,1-Trimethylenäthylen.

Besonders bevorzugte Verbindungen dieser Erfindung sind solche mit E als —C≡C- oder

—$CR_6$=$CR_7$, worin $R_6$ und $R_7$ beide eine Alkylgruppe mit 1-5 C-Atomen darstellen.

Zur Salzbildung mit den freien Säuren ($R_2$=H) sind anorganische und organische Basen geeignet, wie sie dem Fachmann zur Bildung physiologisch verträglicher Salze bekannt sind. Beispielsweise seien genannt : Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostanderivate der allgemeinen Formel I, dadurch gekennzeichnet, daß man in an sich bekannter Weise eine Verbindung der allgemeinen Formel II,

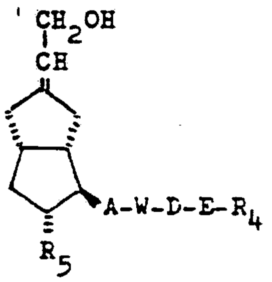

(II)

worin $R_4$, $R_5$, A, W, D und E die obenangegebenen Bedeutungen aufweisen, gegebenenfalls nach Schutz anwesender freier Hydroxygruppen mit einem Halogenessigsäurederivat der allgemeinen Formel III,

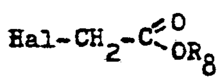

(III)

wobei Hal ein Chlor- oder Bromatom und $R_8$ einen Alkylrest mit 1-4 C-Atomen oder ein Alkalimetall bedeutet, in Gegenwart einer Base veräthert und gegebenenfalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Die Umsetzung der Verbindung der allgemeinen Formel II mit einem Halogenessigsäurederivat der allgemeinen Formel III wird bei Temperaturen von 0 °C bis 100 °C, vorzugsweise 10 °C bis 80 °C, in einem aprotischen Lösungsmittel oder Lösungsmittelgemisch, beispielsweise Dimethylsulfoxid, Dimethylformamid, Tetrahydrofuran usw., vorgenommen. Als Basen kommen die dem Fachmann für Verätherungen bekannten Basen in Frage, beispielsweise Natriumhydrid, Kaliumtert.-butylat, Butyllithium usw.

Die Verseifung der Prostaglandinester wird nach den dem Fachmann bekannten Methoden durchgeführt, wie beispielsweise mit basischen Katalysatoren.

Die Einführung der Estergruppe —$OR_2$ für $R_1$, bei welcher $R_2$ eine Alkylgruppe mit 1-10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. *8,* Seiten 389-394 (1/954)].

Die Einführung des Estergruppe —$OR_2$ für $R_1$, bei welcher $R_2$ Phenyl darstellt, erfolgt nach den dem Fachmann bekannten Methoden. Beispielsweise werden die Carboxyverbindungen mit Phenol und Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform, in Frage. Die Reaktion wird bei Temperaturen zwischen − 30 °C und + 50 °C, vorzugsweise bei + 10 °C, durchgeführt.

Die Prostaglandin-Derivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol gelöst und mindestens

die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0 °C-30 °C nach 15-30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u. a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u. a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 °C und 80 °C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei − 10 °C bis 70 °C, vorzugsweise bei 25 °C.

Die Einführung der Amidgruppe $NHR_3$ für $R_1$ erfolgt nach den dem Fachmann bekannten Methoden. Die Carbonsäuren der allgemeinen Formel I ($R_2$ = H) werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$ = H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen − 30 °C und + 60 °C, vorzugsweise bei 0 °C bis 30 °C.

Eine weitere Möglichkeit für die Einführung der Amidgruppe $NHR_3$ für $R_1$ besteht in der Umsetzung einer 1-Carbonsäure der allgemeinen Formel I ($R_2$ = H), in der freie Hydroxygruppen intermediär geschützt sind, mit Verbindungen der allgemeinen Formel IV,

$$O = C = N—R_3 \qquad (IV)$$

worin $R_3$ die obenangegebene Bedeutung hat.

Die Umsetzung der Verbindung der allgemeinen Formel I ($R_1$ = OH) mit einem Isocyanat der allgemeinen Formel IV erfolgt gegebenenfalls unter Zusatz eines tertiären Amins, wie z. B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen − 80 °C bis 100 °C, vorzugsweise bei 0 °C bis 30 °C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II können beispielsweise hergestellt werden, indem man in an sich bekannter Weise einen Aldehyd der Formel V (DE-OS 28 45 770)

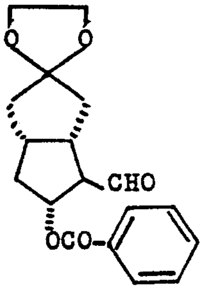

(V)

mit einem Phosphonat der allgemeinen Formel VI

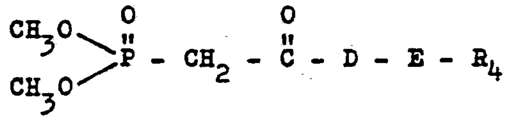

(VI)

worin D, E und $R_4$ die obenangegebene Bedeutung aufweisen, in einer Olefinierungsreaktion zu einem Keton der allgemeinen Formel VII umsetzt.

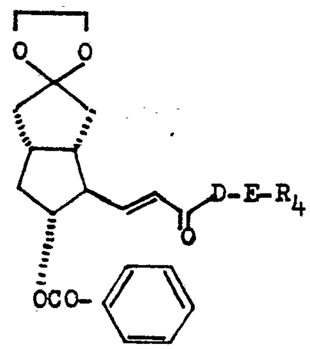

(VII)

Nach Reduktion der Ketogruppe mit Zinkborhydrid oder Natriumborhydrid oder Umsetzung mit Alkylmagnesiumbromid oder Alkyllithium und anschließender Epimerentrennung, sowie gegebenenfalls Hydrierung der Doppelbindung, gelangt man zu den Verbindungen der allgemeinen Formel VIII.

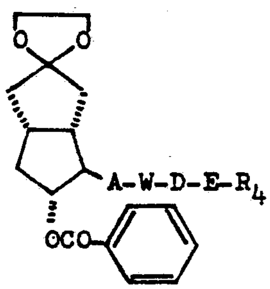

(VIII)

Verseifung der Estergruppe, beispielsweise mit Kaliumcarbonat in Methanol und Ketalspaltung mit wässriger Essigsäure sowie gegebenenfalls funktionelle Abwandlung der freien Hydroxygruppen, beispielsweise durch Verätherung mit Dihydropyran liefert das Keton der allgemeinen Formel IX.

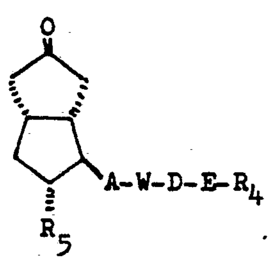

(IX)

Nach Olefinierungsreaktion mit Phosphonoessigsäuretriäthylester oder Phosphonoessigsäuretrimethylester und anschließender Reduktion mit Lithiumaluminiumhydrid erhält man die an der Doppelbindung isomeren Verbindungen der allgemeinen Formel II, die gegebenenfalls getrennt werden können.

Die Herstellung der Phosphonate der allgemeinen Formel VI erfolgt in an sich bekannter Weise durch Umsetzung des Anions von Methylphosphonsäuredimethylester mit einem Ester der allgemeinen Formel X

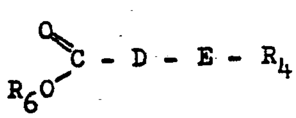

(X)

worin D, E, $R_4$ die obengenannten Bedeutungen aufweisen und $R_6$ eine Alkylgruppe mit 1-5 C-Atomen bedeutet.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Prostaglandin-Analoga besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion, Senkung des systemischen Blutdruckes ohne zugleich Schlagvolumen und koronare Durchblutung zu senken; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion und Zytoprotektion der Magen- und Darmschleimhaut; Zytoprotektion in der Leber und im Pankreas, Senkung des pulmonalen vaskulären Widerstandes und des pulmonalen Blutdruckes, Förderung der Nierendurchblutung, Anwendung anstelle von Heparin oder als Adjuvans bei der Dialyse oder Hämofiltration, Konservierung von Blutplasmakonserven, besonders von Blutplättchenkonserven, Behandlung von Schwangerschaftstoxikose, Erhöhung der zerebralen Durchblutung etc. Die neuen Prostacycline können außerdem in Kombination z. B. mit β-Blockern oder Diuretika Verwendung finden.

Die Dosis der Verbindung ist 1-1 500 µg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 µg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen in Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

Der Einheitsdosisbereich für die Ampulle ist 0,1-0,5 mg, für die Tablette 0,1-1 mg.

## Beispiel 1

(5E)-(16RS)-16-Methyl-3-oxa-18,18,19,19-tetradehydro-6a-carba-prostaglandin-$I_2$

Zu einer Lösung von 530 mg 2-{(E)-(1S,5S,6R,7R)-7-(Tetrahydropyran-2-yloxy)-6-[(E)-(3S,4RS)-4-methyl-3-(tetrahydropyran-2-yloxy)-oct-1-en-6-in-yl]-bicyclo [3.3.0] octan-3-yliden}-äthan-1-ol in 3 ml Tetrahydrofuran fügt man bei 10 °C 0,77 ml einer 1,52 molaren Butyllithiumlösung in Hexan, rührt 5 Minuten, versetzt mit 3 ml Dimethylformamid und 4 ml Dimethylsulfoxyd und fügt anschließend 225 mg Chloressigsäure-Lithiumsalz zu. Man rührt 24 Stunden bei Raumtemperatur, gießt auf Eiswasser, säuert mit 10 %iger Zitronensäurelösung an, extrahiert mit Äther, wäscht den organischen Extrakt einmal mit Sole, trocknet über Magnesiumsulfat und dampft im Vakuum ein. Nach Chromatographie des Rückstandes an Kieselgel erhält man mit Essigester/Isopropanol (8 + 2) 290 mg (5E-(16RS)-16-Methyl-3-oxa-18,18,19,19-tetradehydro-6a-carba-prostaglandin-$I_2$-11,15-bis-(tetrahydropranyläther). Zur Abspaltung der Schutzgruppen rührt man 290 mg des Bis-tetrahydropyranyläthers mit 28 ml einer Mischung aus Essigsäure/Wasser/Tetrahydrofuran (65 + 35 + 10) 16 Stunden bei Raumtemperatur und dampft anschließend im Vakuum ein. Den Rückstand chromatographiert man mit Essigester/Essigsäure (99 + 1) an Kieselgel. Dabei erhält man 105 mg der Titelverbindung als farbloses Öl.

Das Ausgangsmaterial für die obige Titelverbindung wird wie folgt hergestellt:

1a

2-{(E)-(1S,5S,6R,7R)-7-(Tetrahydropyran-2-yloxy)-6-[(E)-(3S,4RS)-4-methyl-3-(tetrahydropyran-2-yloxy)-oct-1-en-6-in-yl]-bicyclo [3.3.0] octan-3-yliden}-äthan-1-ol

Zu einer Lösung von 4,0 g Phosphonoessigsäuretriäthylester in 80 ml Tetrahydrofuran fügt man bei 0 °C 1,73 g Kalium-tert.-butylat, rührt 10 Minuten, versetzt mit einer Lösung aus 4,45 g (1R,5S,6R,7R)-7-(Tetrahydropyran-2-yloxy)-6-[(E)-(3S,4RS)-4-methyl-3-(tetrahydropyran-2-yloxy)-oct-1-en-6-inyl-bicyclo [3.3.0] octan-3-on in 45 ml Toluol und rührt 20 Stunden bei Raumtemperatur. Man verdünnt mit 600 ml Äther, schüttelt einmal mit Wasser, einmal mit 20 %iger Natronlauge, wäscht mit Wasser neutral, trocknet über Magnesiumsulfat und dampft im Vakuum ein. Den Rückstand filtriert man mit Hexan/Äther (3 + 2) über Kieselgel. Dabei erhält man 3,7 g des ungesättigten Esters als farbloses Öl.

IR : 2 945, 2 870, 1 700, 1 655, 970/cm

Man fügt 1,2 g Lithiumaluminiumhydrid portionsweise bei 0 °C zu einer gerührten Lösung von 3,9 g des vorstehend hergestellten Esters in 130 ml Äther und rührt 30 Minuten bei 0 °C. Man zerstört den Reagenzüberschuß durch tropfenweise Zugabe von Essigester, fügt 6 ml Wasser zu, rührt 2 Stunden bei 20 °C, filtriert und dampft im Vakuum ein. Den Rückstand chromatographiert man mit Äther/Hexan (3 + 2) an Kieselgel. Dabei erhält man als unpolarere Verbindung 1,05 g 2-{(Z)-(1S,5S,6R,7R)-7-(Tetrahydropyran-2-yloxy)-6-[(E)-(3S,4RS)-4-methyl-3-(tetrahydropyran-2-yloxy)-oct-1-en-6-in-yl]-bicyclo [3.3.0] octan-3-yliden}-äthan-1-ol und 2,2 g der Titelverbindung als farblose Öle.

IR : 3 605, 3 450, 2 940, 2 865, 1 600, 970/cm

## Beispiel 2

(5Z)-(16RS)-16-Methyl-3-oxa-18,18,19,19-tetradehydro-6a-carba-prostaglandin-$I_2$

In Analogie zu Beispiel 1 erhält man aus 490 mg des nach Beispiel 1a hergestellten Z-konfigurierten Allylalkohols 85 mg der Titelverbindung als farbloses Öl.

IR ($CHCl_3$) : 3 340 (breit), 2 920, 1 722, 1 600, 1 420, 966/cm

## Beispiel 3

(5E)-(16RS)-16,20-Dimethyl-3-oxa-18,18,19,19-tetradehydro-6a-carba-prostaglandin-$I_2$

In Analogie zu Beispiel 1 erhält man aus 0,5 g 2-{(E)-(1S,5S,6R,7R)-7-(Tetrahydropyran-2-yloxy)-6-[(E)-(3S,4RS)-4-methyl-3-(tetrahydropyran-2-yloxy)-non-1-en-6-in-yl]-bicyclo [3.3.0] octan-3-yliden}-äthan-1-ol und 220 mg Chloressigsäure-Lithiumsalz 260 mg (5E)-(16RS)-16,20-Dimethyl-3-oxa-18,18,19,19-tetradehydro-6a-carbaprostaglandin-$I_2$-11,15-bis-(tetrahydropyranyläther). Nach Abspaltung der Schutzgruppen erhält man 90 mg der Titelverbindung als farbloses Öl.

IR : 3 400 (breit), 2 920, 1 721, 1 600, 1 420, 966/cm

Das Ausgangsmaterial für die obige Titelverbindung wird wie folgt hergestellt :

### 3a

(1R,5S,6R,7R)-3,3-Äthylendioxy-7-benzoyloxy-6-[(E)-(3S,4RS)-3-hydroxy-4-methyl-non-1-en-6-inyl]-bicyclo [3.3.0] octan

Zu einer Suspension von 1,46 g Natriumhydrid (55 %ige Suspension in Öl) in 130 ml Dimethoxyäthan (DME) tropft man bei 0 °C eine Lösung aus 9,02 g 3-Methyl-2-oxo-oct-5-inyl-phosphonsäure-dimethylester in 67 ml DME und rührt 1 Stunde bei 0 °C. Anschließend versetzt man bei − 20 °C mit einer Lösung aus 9,4 g (1R,5S,6R,7R)-3,3-Äthylendioxy-7-benzoyloxy-6-formyl-bicyclo [3.3.0]-octan in 130 ml DME, rührt 1,5 Stunden bei − 20 °C, gießt auf 600 ml gesättigte Ammoniumchloridlösung und extrahiert dreimal mit Äther. Man wäscht den organischen Extrakt mit Wasser neutral, trocknet über Magnesiumsulfat und dampft im Vakuum ein. Nach Chromatographie des Rückstandes and Kieselgel erhält man mit Äther/Hexan (1 + 1) 9,1 g des α,β-ungesättigten Ketons als Öl.

Zu einer Lösung von 9,1 g des Ketons in 300 ml Methanol fügt man bei − 40 °C portionsweise 5,2 g Natriumborhydrid und rührt 1 Stunde bei − 40 °C. Anschließend verdünnt man mit Äther, wäscht mit Wasser neutral, trocknet über Magnesiumsulfat und dampft im Vakuum ein. Durch Säulenchromatographie an Kieselgel mit Äther/Hexan (7 + 3) erhält man zunächst 3,9 g der Titelverbindung (PG-Nomenklatur : 15α-Hydroxy) sowie als polarere Komponente 3,2 g der isomeren 15 β-Hydroxy-verbindung.

IR : 3 600, 3 400 (breit), 2 942, 1 711, 1 603, 1 588, 1 276, 968, 947/cm

### 3b

(1R,5S,6R,7R)-7-(Tetrahydropyran-2-yloxy)-6-[(E)-(3S,4RS)-3-(tetrahydropyran-2-yloxy)-4-methyl-non-1-en-6-inyl]-bicyclo [3.3.0] octan-3-on

Eine Mischung aus 3,6 g des nach Beispiel 3a hergestellten α-Alkohols und 1,4 g Kaliumcarbonat in 120 ml Methanol rührt man 16 Stunden bei Raumtemperatur unter Argon. Anschließend engt man im Vakuum ein, verdünnt mit Äther und wäscht mit Sole neutral. Man trocknet über Magnesiumsulfat und

dampft im Vakuum ein. Den Eindampfrückstand rührt man 16 Stunden bei Raumtemperatur mit 75 ml einer Mischung aus Essigsäure/Wasser/Tetrahydrofuran (65 + 35 + 10) und dampft anschließend im Vakuum ein. Nach Filtration des Rückstandes über Kieselgel erhält man mit Essigester/Hexan (7 + 3) 2,2 g des Ketons als Öl.

Eine Lösung aus 2,2 g des Ketons, 2,4 ml Dihydropyran und 23 mg p-Toluolsulfonsäure in 75 ml Methylenchlorid rührt man 30 Minuten bei 0 °C. Anschließend verdünnt man mit Äther, schüttelt mit verd. Natriumbicarbonat-Lösung, wäscht mit Wasser neutral, trocknet über Magnesiumsulfat und dampft im Vakuum ein. Man erhält 3,4 g des Bis-tetrahydropyranyläthers, der ohne Reinigung verwendet wird.

IR : 2 960, 2 865, 1 738, 970/cm

3c
2-{(E)-(1S,5S,6R,7R)-7-(Tetrahydropyran-2-yloxy)-6-[(E)-(3S,4RS)-4-methyl-3-(tetrahydropyran-2-yloxy)-non-1-en-6-in-yl]-bicyclo [3.3.0] octan-3-yliden}-äthan-1-ol

In Analogie zu Beispiel 1a erhält man aus 3,3 g des nach Beispiel 3b hergestellten Ketons nach chromatographischer Isomerentrennung als unpolarere Verbindung 720 mg 2-{(Z)-(1S,5S,6R,7R)-7-(Tetrahydropyran-2-yloxy)-6-[(E)-(3S,4RS)-4-methyl-3-(tetrahydropyran-2-yloxy)-non-1-en-6-in-yl]-bicyclo [3.3.0] octan-3-yliden}-äthan-1-ol und 1,6 g der Titelverbindung als farbloses Öl.

IR : 3 600, 3 430, 2 942, 2 863, 1 600, 972/cm

## Beispiel 4

(5Z)-(16RS)-16,20-Dimethyl-3-oxa-18,18,19,19-tetradehydro-6a-carba-prostaglandin-$I_2$

In Analogie zu Beispiel 1 erhält man aus 0,65 g des nach Beispiel 3c hergestellten Z-konfigurierten Allylalkohols 120 mg der Titelverbindung als farbloses Öl.

IR : 3 320 (breit), 2 925, 1 720, 1 600, 1 420, 968/cm

## Beispiel 5

(5E)-(16RS)-16,20-Dimethyl-3-oxa-19,19,20,20-tetradehydro-6a-carba-prostaglandin-$I_2$

In Analogie zu Beispiel 1 erhält man aus 0,75 g 2-{(E)-(1S,5S,6R,7R)-7-(Tetrahydropyran-2-yloxy)-6-[(E)-(3S,4RS)-4-methyl-3-(tetrahydropyran-2-yloxy)-non-1-en-7-inyl]-bicyclo [3.3.0] octan-3-yliden}-äthan-1-ol und 330 mg Chloressigsäure-Lithiumsalz 420 mg (5E)-(16RS)-16,20-Dimethyl-3-oxa-19,19,20,20-tetradehydro-6a-carba-prostaglandin-$I_2$-11,15-bis-(tetrahydropyranyläther). Nach Abspaltung der Schutzgruppen erhält man 180 mg der Titelverbindung als farbloses Öl.

IR : 3 410 (breit), 2 925, 1 722, 1 601, 1 420, 965/cm

Das Ausgangsmaterial für die obige Titelverbindung wird wie folgt hergestellt :

5a
3-Methyl-2-oxo-oct-6-inyl-phosphonsäure-dimethylester

Zu einer Lösung von 40 g 3-Pentin-1-ol in 240 ml Pyridin fügt man 109 g p-Toluolsulfonsäurechlorid und rührt 48 Stunden bei 0 °C. Anschließend fügt man 30 ml Wasser zu, rührt 2 Stunden und verdünnt mit Äther. Man schüttelt nacheinander mit 5 %iger Schwefelsäure, mit Wasser, mit 5 %iger Natriumbicarbonatlösung, wäscht mit Wasser neutral, trocknet über Magnesiumsulfat und dampft im Vakuum ein. Den Rückstand kristallisiert man aus Hexan/Äther um. Dabei erhält man 80 g des Tosylats (Fp. 43 °C).

Zu einer Suspension von 8,7 g Natriumhydrid/55 %ige Suspension in Öl) fügt man 34 g Methylmalonester und kocht 4 Stunden unter Rückfluß. Anschließend fügt man bei Raumtemperatur 35 g 1-Tosyloxy-3-pentin und 130 ml DME zu und erhitzt 8 Stunden unter Rückfluß. Man neutralisiert mit Essigsäure, versetzt mit 130 ml Wasser, extrahiert mit Äther, schüttelt dreimal mit Wasser, trocknet über Magnesiumsulfat und dampft im Vakuum ein. Den Rückstand reinigt man durch Vakuumdestillation bei 12 Torr. Bei 150-156 °C erhält man 24 g des alkylierten Methylmalonsäureesters, den man in 160 ml Dimethylsulfoxid und 1,5 ml Wasser 6 h mit 7,5 g Lithiumchlorid unter Rückfluß erhitzt. Anschließend gießt man auf 400 ml Eiswasser, extrahiert mit Äther, schüttelt den organischen Extrakt zweimal mit Wasser, trocknet über Magnesiumsulfat und engt im Vakuum ein. Die Destillation des Rückstandes liefert bei 92 °C und 12 Torr 14 g 2-Methyl-hept-5-insäure-äthylester als farblose Flüssigkeit.

Zu einer Lösung von 18,6 g Methanphosphonsäuredimethylester in 280 ml Tetrahydrofuran tropft man 73 ml einer 1,7 molaren Butyllithiumlösung in Hexan bei − 70 °C, rührt 15 Minuten und fügt langsam eine Lösung von 10,5 g 2-Methyl-hept-5-insäureäthylester in 48 ml Tetrahydrofuran zu. Man rührt 4 Stunden bei − 70 °C, neutralisiert mit Essigsäure und dampft im Vakuum ein. Die Destillation des Rückstandes im Vakuum bei 0, 6 Torr und 130 °C liefert 10,8 g der Titelverbindung als farblose Flüssigkeit.

5b
(1R,5S,6R,7R)-3,3-Äthylendioxy-7-benzoyloxy-6-[(E)-(3S,4RS)-3-hydroxy-4-methyl-non-1-en-7-inyl]-bicyclo [3.3.0] octan

In Analogie zu Beispiel 3a erhält man aus 12,2 g (1R,5S,6R,7R)-3,3-Äthylendioxy-7-benzoyloxy-6-formyl-bicyclo [3.3.0] octan und 11,9 g des nach Beispiel 5a hergestellten Phosphonats 14,5 g des ungesättigten Ketons, welches durch Reduktion mit 8,2 g Natriumborhydrid in 5,3 g der Titelverbindung überführt wird.
IR : 3 600, 3 410, 2 940, 1 712, 1 602, 1 588, 1 277, 970, 948/cm

5c
(1R,5S,6R,7R)-7-(Tetrahydropyran-2-yloxy)-6-[(E)-(3S,4RS)-3-(tetrahydropyran-2-yloxy)-4-methyl-non-1-en-7-inyl]-bicyclo [3.3.0] octan-3-on

In Analogie zu Beispiel 3b erhält man aus 5,3 g des nach Beispiel 5b hergestellten α-Alkohols 5,4 g des Bis-tetrahydropyranyläthers als farbloses Öl.
IR : 2 963, 2 866, 1 737, 968/cm

5d
2-{(E)-(1S,5S,6R,7R)-7-(Tetrahydropyran-2-yloxy)-6-[(E)-(3S,4RS)-4-methyl-3-(tetrahydropyran-2-yloxy)-non-1-en-7-inyl]-bicyclo [3.3.0] octan-3-yliden}-äthan-1-ol

In Analogie zu Beispiel 1a erhält man aus 5,3 g des nach Beispiel 5c hergestellten Ketons nach chromatographischer Isomerentrennung als unpolarere Verbindung 1,4 g 2-{(Z)-(1S,5S,6R,7R)-7-(Tetrahydropyran-2-yloxy)-6-[(E)-(3S,4RS)-4-methyl-3-(tetrahydropyran-2-yloxy)-non-1-en-7-inyl]-bicyclo [3.3.0] octan-3-yliden}-äthan-1-ol und 2,9 g der Titelverbindung als farbloses Öl.
IR : 3 610, 3 400, 2 940, 2 862, 1 600, 970/cm

Beispiel 6

(5Z)-(16RS)-16,20-Dimethyl-3-oxa-19,19,20,20-tetradehydro-6a-carba-prostaglandin-$I_2$

In Analogie zu Beispiel 1 erhält man aus 810 mg des nach Beispiel 5d hergestellten Z-konfigurierten Allylalkohols 180 mg der Titelverbindung als farbloses Öl.
IR : 3 430 (breit), 2 030, 1 721, 1 600, 1 425, 968/cm

Beispiel 7

(5E)-(15RS)-15-Methyl-3-oxa-18,18,19,19-tetradehydro-6a-carba-prostaglandin-$I_2$

In Analogie zu Beispiel 1 erhält man aus 0,45 g 2-{(E)-(1S,5S,6R,7R)-7-(Tetrahydropyran-2-yloxy)-6-[(E)-(3RS)-3-methyl-3-(tetrahydropyran-2-yloxy)-oct-1-en-6-in-yl]-bicyclo [3.3.0] octan-3-yliden}-äthan-1-ol und 210 mg Chloressigsäure-Lithiumsalz 270 mg (5E)-(15RS)-15-methyl-3-oxa-18,18,19,19-tetradehydro-6a-carba-prostaglandin-$I_2$-11,15-bis-tetrahydropyranyläther. Nach Abspaltung der Schutzgruppen erhält man 108 mg der Titelverbindung als farbloses Öl.
IR : 3 410 (breit), 2 945, 1 720, 1 600, 968/cm

Das Ausgangsmaterial für die obige Titelverbindung wird wie folgt hergestellt :

7a
2-{(E)-(1S,5S,6R,7R)-7-(Tetrahydropyran-2-yloxy)-6-[(E)-(3RS)-3-methyl-3-methyl-3-(tetrahydropyran-2-yloxy)-oct-1-en-6-inyl]-bicyclo [3.3.0] octan-3-yliden}-äthan-1-ol

In Analogie zu Beispiel 1a erhält man aus 1,7 g (1R,5S,6R,7R)-7-(Tetrahydropyran-2-yloxy)-6-[(E)-(3RS)-3-methyl-3-(tetrahydropyran-2-yloxy)-oct-1-en-6-inyl]-bicyclo [3.3.0] octan-3-on nach chromatographischer Isomerentrennung als unpolarere Verbindung 320 mg 2-{(Z)-(1S,5S,6R,7R)-7-(Tetrahydropyran-2-yloxy)-6-[(E)-(3RS)-3-methyl-3-(tetrahydropyran-2-yloxy)-oct-1-en-6-inyl]-bicyclo [3.3.0] octan-3-yliden}-äthan-1-ol und 950 mg der Titelverbindung als farbloses Öl.
IR : 3 600, 3 400, 2 945, 2 865, 1 600, 970/cm

Beispiel 8

(5E)-3-Oxa-18,18,19,19-tetradehydro-6a-carba-prostaglandin-$I_2$

In Analogie zu Beispiel 1 erhält man aus 0,92 g 2-{(E)-(1S,5S,6R,7R)-7-(Tetrahydropyran-2-yloxy)-6-[(E)-3-(tetrahydropyran-2-yloxy)-oct-1-en-6-in-yl]-bicyclo [3.3.0] octan-3-yliden)-äthan-1-ol und 430 mg Chloressigsäure-Lithiumsalz 510 mg (5E)-3-Oxa-18,18,19,19-tetradehydro-6a-carba-prostaglandin-$I_2$-11,15-bis-tetra-hydropyranyläther. Nach anschließender Abspaltung der Schutzgruppen erhält man 245 g der Titelverbindung als farbloses Öl.

IR : 3 400 (breit), 2 225, 1 722, 1 600, 972/cm

Das Ausgangsmaterial für die obige Titelverbindung wird wie folgt hergestellt :

8a

2-{(E)-(1S,5S,6R,7R)-7-(Tetrahydropyran-2-yloxy)-6-[(E)-3-(tetrahydropyran-2-yloxy)-oct-1-en-6-in-yl]-bicyclo [3.3.0] octan-3-yliden}-äthan-1-ol

In Analogie zu Beispiel 1a erhält man aus 3,35 g (1R,5S,6R,7R)-7-(Tetrahydropyran-2-yloxy)-6-[(E)-3-tetrahydropyran-2-yloxy)-oct-1-en-6-inyl]-bicyclo [3.3.0.] octan-3-on nach chromatographischer Isomerentrennung als unpolarere Verbindung 820 mg 2-{(Z)-(1S,5S,6R,7R)-7-(Tetrahydropyran-2-yloxy)-6-[(E)-3-(tetrahydropyran-2-yloxy)-oct-1-en-6-in-yl]-bicyclo [3.3.0] octan-3-yliden}-äthan-1-ol und 1,9 g der Titelverbindung als farbloses Öl.

IR : 3 600, 3 405, 2 943, 2 865, 1 600, 968/cm

Beispiel 9

(5E)-(16RS)-16,19-Dimethyl-3-oxa-18,19-didehydro-6a-carba-prostaglandin-$I_2$

In Analogie zu Beispiel 1 erhält man aus 0;97 g 2-{(E)-(1S,5S,6R,7R)-7-(Tetrahydropyran-2-yloxy)-6-(1E)-(3S,4RS)-4,7-dimethyl-3-(tetrahydropyran-2-yloxy)-oct-1,6-dienyl]-bicyclo [3.3.0] octan-3-yliden-äthan-1-ol und 450 mg Chloressigsäure-Lithiumsalz 490 mg (5E)-(16RS)-16,19-Dimethyl-3-oxa-18,19-didehydro-6a-carba-prostaglandin-$I_2$-11,15-bis-tetrahydropyranyläther. Die anschließende Abspaltung der Schutzgruppen liefert 160 mg der Titelverbindung als Öl.

IR : 3 410 (breit), 2 925, 1 720, 1 600, 965/cm

Das Ausgangsmaterial für die obige Titelverbindung wird wie folgt hergestellt :

9a

(1R,5S,6R,7R)-3,3-Äthylendioxy-7-benzoyloxy-6-[(1E)-(3S,4RS)-3-hydroxy-4,7-dimethyl-oct-1,6-dienyl]-bicyclo [3.3.0] octan

In Analogie zu Beispiel 3a erhält man aus 6,5 g (1R,5S,6R,7R)-3,3-Äthylendioxy-7-benzoyloxy-6-formyl-bicyclo [3.3.0] octan und 6 g 2-Oxo-3,6-dimethyl-hept-5-enyl-phosphonsäure-dimethylester 6,3 g des ungesättigten Ketons, welches durch Reduktion mit 4 g Natriumborhydrid in 2,7 g der Titelverbindung überführt wird.

IR : 3 600, 3 420, 2 945, 1 713, 1 602, 1 587, 1 278, 972, 948/cm

9b

(1R,5S,6R,7R)-7-(Tetrahydropyran-2-yloxy)-6-[(1E)-(3S,4RS)-4,7-dimethyl-3-(tetrahydropyran-2-yloxy)-oct-1,6-dienyl]-bicyclo [3.3.0] octan-3-on

In Analogie zu Beispiel 3b erhält man aus 2,7 g des nach Beispiel 9a hergestellten α-Alkohols 2,6 g der Titelverbindung als farbloses Öl.

IR : 2 965, 2 865, 1 738, 965/cm

9c

2-{(E)-(1S,5S,6R,7R)-7-(Tetrahydropyran-2-yloxy)-6-[(1E)-[3S,4RS)-4,7-dimethyl-3-(tetrahydropyran-2-yloxy)-oct-1,6-dienyl]-bicyclo [3.3.0] octan-3-yliden}-äthan-1-ol

In Analogie zu Beispiel 1a erhält man aus 2,6 g des nach Beispiel 9b hergestellten Ketons nach chromatographischer Isomerentrennung als unpolarere Verbindung 0,65 g 2-{(Z)-(1S,5S,6R,7R)-7-(Tetrahydropyran-2-yloxy)-6-[(1E)-(3S,4RS)-4,7-dimethyl-3-(tetrahydropyran-2-yloxy)-oct-1,6-dienyl]-bicyclo [3.3.0] octan-3-yliden}-äthan-1-ol und 1,4 g der Titelverbindung als farbloses Öl.

IR : 3 600, 3 400 (breit), 2 945, 2 860, 1 600, 968/cm

Beispiel 10

(5E)-(16RS)-16-Methyl-3-oxa-18,18,19,19-tetradehydro-6a-carba-prostaglandin-$I_2$-methylester

Man versetzt eine Lösung von 150 mg der nach Beispiel 1 hergestellten Säure in 10 ml Methylenchlorid bei 0 °C tropfenweise mit einer ätherischen Diazomethanlösung bis zur bleibenden Gelbfärbung. Nach Eindampfen der Lösung im Vakuum filtriert man den Rückstand mit Methylenchlorid/Isopropanol

(95 + 5) über Kieselgel und erhält 120 mg des Methylesters als farbloses Öl.
IR : 3 600, 3 400, 2 960, 1 740, 1 600, 972/cm

Beispiel 11

(5E)-(16RS)-16-Methyl-3-oxa-18,18,19,19-tetradehydro-N-methansulfonyl-6a-carba-prostaglandin-$I_2$-carboxamid

Eine Lösung von 360 mg der nach Beispiel 1 hergestellten Säure in 8 ml Dimethylformamid wird bei 0 °C mit 160 mg Chlorameisensäureisobutylester und 120 mg Triäthylamin versetzt. Nach 30 Minuten werden 480 mg des Natriumsalzes von Methylsulfonamid (hergestellt aus Methylsulfonamid und Natriummethylat) und 2 ml Hexamethylphosphorsäuretriamid zugesetzt und 3 Stunden bei 20 °C gerührt. Anschließend gibt man das Reaktionsgemisch auf Citratpuffer (pH 5), extrahiert mehrere Male mit Essigester, wäscht die organische Phase mit Sole, trocknet über Magnesiumsulfat und dampft im Vakuum ein. Nach Chromatographie des Rückstandes an Kieselgel mit Methylenchlorid/Isopropanol erhält man 160 mg der Titelverbindung als Öl.
IR : 3 600, 3 400 (breit), 1 730, 1 600, 970/cm

Beispiel 12

(5E)-(16RS)-16-Methyl-3-oxa-18,18,19,19-tetradehydro-6a-carba-prostaglandin-$I_2$-acetylamid

Zu einer Lösung von 500 mg (5E)-(16RS)-16-Methyl-3-oxa-18,18,19,19-tetradehydro-6a-carba-prostaglandin-$I_2$-11,15-bis-(tetrahydropyranyläther) in 15 ml Acetonitril gibt man bei 25 °C 130 mg Triäthylamin, kühlt auf 0 °C und tropft eine Lösung von 95 mg Acetylisocyanat in 10 ml Acetonitril zu. Man rührt 2 Stunden bei 25 °C, engt im Vakuum ein, verdünnt mit 100 ml Wasser, stellt durch Zugabe von 1 N Salzsäure auf pH 5 ein, extrahiert mit Äther, wäscht die organische Phase mit Sole, trocknet über Magnesiumsulfat und dampft im Vakuum ein. Zur Abspaltung der Schutzgruppen rührt man den Rückstand mit 15 ml Eisessig/Wasser/Tetrahydrofuran (65/25/10) über Nacht bei 30 °C und dampft im Vakuum zur Trockne. Der Rückstand wird an Kieselgel mit Methylenchlorid/1 % Isopropylalkohol chromatographiert. Man erhält 205 mg der Titelverbindung als Öl.
IR : 3 600, 3 400, 1 708, 976/cm

Beispiel 13

(5E)-(16RS)-16-Methyl-3-oxa-18,18,19,19-tetradehydro-6a-carba-prostaglandin-$I_2$-carboxamid

200 mg (5E)-(16RS)-Methyl-3-oxa-18,18,19,19-tetradehydro-6a-carba-prostaglandin-$I_2$ werden in 5 ml Tetrahydrofuran gelöst und bei 0 °C mit 80 mg Triäthylamin und 90 mg Chlorameisensäureisobutylester versetzt. Nach 1 Stunde wird bei 0 °C 10 Minuten Ammoniakgas eingeleitet, dann 1 Stunde bei 25 °C belassen. Anschließend wird mit 50 ml Wasser verdünnt, dreimal mit je 30 ml Methylenchlorid extrahiert, die vereinigten Extrakte mit 20 ml Sole geschüttelt, über Magnesiumsulfat getrocknet und im Vakuum eingedampft. Man reinigt durch Chromatographie an Kieselgel mit Chloroform/Essigester (8 + 2) und erhält 150 mg der Titelverbindung als Öl.
IR : 3 600, 3 540, 3 410, 2 960, 1 670, 978/cm

Beispiel 14

(5E)-(16RS)-16-Methyl-3-oxa-18,18,19,19-tetradehydro-6a-carba-prostaglandin-$I_2$trishydroxymethylaminomethansalz

Zu einer Lösung von 200 mg (5E)-(16RS)-16-Methyl-3-oxa-18,18,19,19-tetradehydro-6a-carba-prostaglandin-$I_2$ in 35 ml Acetonitril gibt man bei 65 °C eine Lösung von 60 mg Tris(hydroxymethyl)-aminomethan in 0,2 ml Wasser. Man läßt unter Rühren abkühlen, dekantiert nach 16 Stunden vom Lösungsmittel und trocknet den Rückstand im Vakuum. Man isoliert 190 mg der Titelverbindung als zähes Öl.

Beispiel 15

Zusammensetzung pro Ampulle :
0,5 mg (5E)-(16RS)-16-Methyl-3-oxa-18,18,19,19-tetradehydro-6a-carba-prostaglandin-$I_2$
8,9 mg NaCl
1,212 mg Tromethamol
0,01 ml Äthanol (96 %ig)
mit 0,01 n HCl-Lösung auf pH 8,3 eingestellt ad 1 ml mit Wasser für Injektionszwecke

**Ansprüche** (für die Vertragsstaaten : BE, CH, DE, FR, GB, IT, LI, LU, NL, SE)

1. Carbacyclinderivate der allgemeinen Formel I

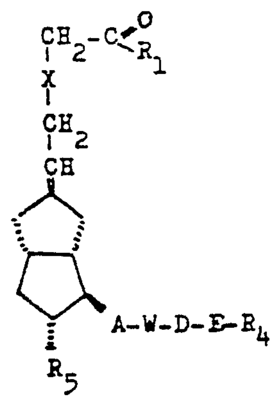

worin

$R_1$ den Rest $OR_2$, wobei $R_2$ Wasserstoff, einen Alkylrest mit 1-10 C-Atomen, der durch Fluor, Chlor, Brom, Phenyl, Dimethylamino, Diethylamino, Methoxy oder Ethoxy substituiert sein kann, einen Cycloalkylrest mit 5-6 C-Atomen, der durch einen Alkylrest mit 1-4 C-Atomen substituiert sein kann, einen Phenylrest oder die Reste 2- oder 3-Furyl, 2- oder 3-Thienyl oder 2-3- oder 4-Pyridyl bedeuten kann, oder den Rest $NHR_3$ mit $R_3$ in der Bedeutung einer Carbonsäure oder Sulfonsäure mit 1-15 C-Atomen oder eines Wasserstoffatoms,

X Sauerstoff

A eine $—CH_2—CH_2—$, trans-CH=CH- oder —C≡C-Gruppe,

W eine freie oder funktionell abgewandelte Hydroxymethylengruppe oder eine freie oder funktionell abgewandelte

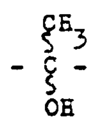

Gruppe, wobei die OH-Gruppe α- oder β-ständig sein kann,

D eine geradkettige oder verzweigte, gesättigte oder ungesättigte Alkylengruppe mit 1-10 C-Atomen, die durch Fluoratome, 1,2-Methylen oder 1,1-Trimethylen substituiert sein kann,

E eine —C≡C-Gruppe oder eine $—CR_6=CR_7$-Gruppe darstellt, wobei $R_6$ und $R_7$ ein Wasserstoffatom oder eine Alkygruppe mit 1-5 C-Atomen bedeuten,

$R_4$ eine Alkylgruppe mit 1-10 C-Atomen, eine Cycloalkylgruppe mit 5-6 C-Atomen, Phenyl oder die Reste 2- oder 3-Furyl, 2-oder 3-Thienyl- oder 2-, 3- oder 4-Pyridyl,

$R_5$ eine freie oder funktionell abgewandelte Hydroxygruppe,

und, falls $R_2$ die Bedeutung eines Wasserstoffatoms hat, deren Salze mit physiologisch verträglichen Basen bedeuten.

2. (5E)-(16RS)-16-Methyl-3-oxa-18,18,19,19-tetradehydro-6a-carba-prostaglandin-$I_2$
3. (5Z)-(16RS)-16-Methyl-3-oxa-18,18,19,19-tetradehydro-6a-carba-prostaglandin-$I_2$
4. (5E)-(16RS)-16,20-Dimethyl-3-oxa-18,18,19,19-tetradehydro-6a-carba-prostaglandin-$I_2$
5. (5Z)-(16RS)-16,20-Dimethyl-3-oxa-18,18,19,19-tetradehydro-6a-carba-prostaglandin-$I_2$
6. (5E)-(16RS)-16,20-Dimethyl-3-oxa-19,19,20,20-tetradehydro-6a-carba-prostaglandin-$I_2$
7. (5Z)-(16RS)-16,20-Dimethyl-3-oxa-19,19,20,20-tetradehydro-6a-carba-prostaglandin-$I_2$
8. (5E)-(15RS)-15-Methyl-3-oxa-18,18,19,19-tetradehydro-6a-carba-prostaglandin-$I_2$
9. (5E)-3-Oxa-18,18,19,19-tetradehydro-6a-carba-prostaglandin-$I_2$
10. (5E)-(16RS)-16,19-Dimethyl-3-oxa-18,19-didehydro-6a-carba-prostaglandin-$I_2$
11. (5E)-(16RS)-Methyl-3-oxa-18,18,19,19-tetradehydro-6a-carba-prostaglandin-$I_2$-methylester
12. (5E)-(16RS)-16-Methyl-3-oxa-18,18,19,19-tetradehydro-N-methansulfonyl-6a-carba-prostaglandin-$I_2$-carboxamid
13. (5E)-(16RS)-16-Methyl-3-oxa-18,18,19,19-tetradehydro-6a-carba-prostaglandin-$I_2$-acetylamid
14. (5E)-(16RS)-16-Methyl-3-oxa-18,18,19,19-tetradehydro-6a-carba-prostaglandin-$I_2$-carboxamid
15. (5E)-(16RS)-16-Methyl-3-oxa-18,18,19,19-tetradehydro-6a-carba-prostaglandin-$I_2$-trishydroxymethylaminomethansalz
16. Verfahren zur Herstellung der Carbacyclinderivate der allgemeinen Formel I, dadurch gekennzeichnet, daß man in an sich bekannter Weise eine Verbindung der allgemeinen Formel II,

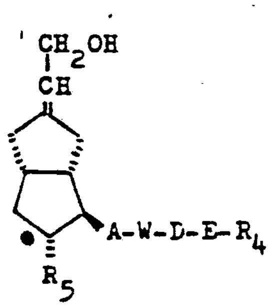

(II)

worin $R_4$, $R_5$, A, W, D und E die obenangegebenen Bedeutungen aufweisen, gegebenenfalls nach Schutz anwesender freier Hydroxygruppen mit einem Halogenessigsäurederivat der allgemeinen Formel III,

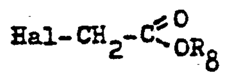

(III)

wobei Hal ein Chlor- oder Bromatom und $R_8$ einen Alkylrest mit 1-4 C-Atomen oder ein alkalimetall bedeutet, in Gegenwart einer base veräthert und gegebenefalls anschließend in beliebiger Reihenfolge Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

17. Arzneimittel, bestehend aus einer oder mehreren Verbindungen gemäß Anspruch 1 und üblichen Hilfs- und Trägerstoffen.

**Anspruch** (für den Vertragsstaat AT)

Verfahren zur Herstellung der Carbacyclinderivate der allgemeinen Formel I

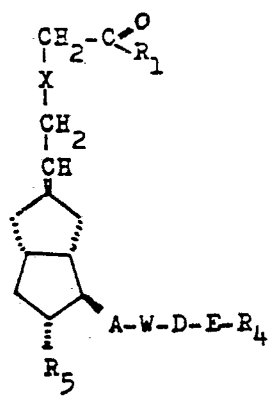

(I)

worin

$R_1$ den Rest $OR_2$, wobei $R_2$ Wasserstoff, einen Alkylrest mit 1-10 C-Atomen, der durch Fluor, Chlor, Brom, Phenyl, Dimethylamino, Diethylamino, Methoxy oder Ethoxy substituiert sein kann, einen Cycloalkylrest mit 5-6 C-Atomen, der durch einen Alkylrest mit 1-4 C-Atomen substituiert sein kann, einen Phenylrest oder die Reste 2- oder 3-Furyl, 2- oder 3-Thienyl oder 2-3- oder 4-Pyridyl bedeuten kann, oder den Rest $NHR_3$ mit $R_3$ in der Bedeutung einer Carbonsäure oder Sulfonsäure mit 1-15 C-Atomen oder eines Wasserstoffatoms,

X Sauerstoff

A eine $-CH_2-CH_2-$, trans-CH=CH- oder $-C\equiv C$-Gruppe,

W eine freie oder funktionell abgewandelte Hydroxymethylengruppe oder eine freie oder funktionell abgewandelte

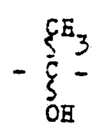

Gruppe, wobei die OH-Gruppe α- oder β-ständig sein kann,

D eine geradkettige oder verzweigte, gesättigte oder ungesättigte Alkylengruppe mit 1-10 C-Atomen, die durch Fluoratome, 1,2-Methylen oder 1,1-Trimethylen substituiert sein kann,

E eine —C≡C-Gruppe oder eine —$CR_6$=$CR_7$-Gruppe darstellt, wobei $R_6$ und $R_7$ ein Wasserstoffatom oder eine Alkylgruppe mit 1-5 C-Atomen bedeuten,

$R_4$ eine Alkylgruppe mit 1-10 C-Atomen, eine Cycloalkylgruppe mit 5-6 C-Atomen, Phenyl oder die Reste 2- oder 3-Furyl, 2- oder 3-Thienyl- oder 2-, 3- oder 4-Pyridyl

$R_5$ eine freie oder funktionell abgewandelte Hydroxygruppe,

und, falls $R_2$ die Bedeutung eines Wasserstoffatoms hat, deren Salze mit physiologisch verträglichen Basen bedeuten, dadurch gekennzeichnet, daß man in an sich bekannter Weise eine Verbindung der allgemeinen Formel II

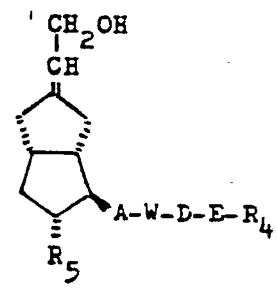

(II)

worin $R_4$, $R_5$, A, W, D und E obenangegebenen Bedeutungen aufweisen, gegebenenfalls nach Schutz anwesender freier Hydroxygruppen mit einem Halogenessigsäurederivat der allgemeinen Formel III,

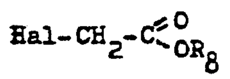

(III)

wobei Hal ein Chlor- oder Bromatom und $R_8$ einen Alkylrest mit 1-4 C-Atomen oder ein Alkalimetall bedeutet, in Gegenwart einer Base veräthert und gegebenenfalls anschließend in beliebiger Reihenfolge Isomere trennt, und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

**Claims** (for the Contracting States : BE, CH, DE, FR, GB, IT, LI, LU, NL, SE)

1. Carbacyclin derivatives of general formula I

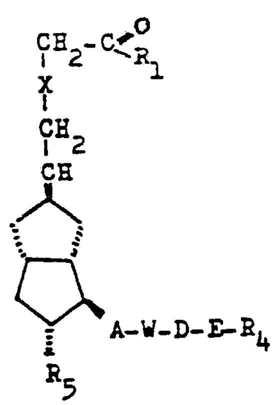

(I)

wherein

$R_1$ is the group $OR_2$, wherein $R_2$ is hydrogen, alkyl of 1-10 carbon atoms, (which can be substituted by fluorine, chlorine, bromine, phenyl, dimethylamino, diethylamino, methoxy or ethoxy), cycloalkyl of 5-6 carbon atoms, (which can be substituted by alkyl of 1-4 carbon atoms), phenyl, 2- or 3-furyl, 2- or 3-thienyl or 2- 3- or 4-pyridyl, or $R_1$ is the group $NHR_3$, where $R_3$ is a carboxylic acid or sulphonic acid group of 1-15 carbon atoms, or is hydrogen ;

X is oxygen ;

A is a $—CH_2—CH_2—$, trans-CH=CH- of —C≡C-group ;

W is a free or functionally modified hydroxymethylene group, or a free or functionally modified

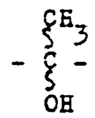

group, in which the OH-group can be in the alpha- or beta position ;

D is a straight or branched chain, saturated or unsaturated alkylene group of 1-10 carbon atoms, which can be substituted by fluorine, 1,2-methylene or 1,1-trimethylene

E is a —C≡C-group or a $—CR_6=CR_7$-group, wherein $R_6$ and $R_7$ are hydrogen or alkyl of 1-5 carbon atoms

$R_4$ is alkyl of 1-10 carbon atoms, cycloalkyl of 5-6 carbon atoms, phenyl, 2- or 3-furyl, 2- or 3-thienyl of 2-, 3- or 4-pyridyl ;

$R_5$ is a free or functionally modified hydroxy group,

and when $R_2$ is hydrogen, salts of these compounds with physiologically compatible bases.

2. (5E)-(16RS)-16-Methyl-3-oxa-18,18,19,19-tetradehydro-6a-carba-prostaglandin-$I_2$

3. (5Z)-(16RS)-16-Methyl-3-oxa-18,18,19,19-tetradehydro-6a-carba-prostaglandin-$I_2$

4. (5E)-(16RS)-16,20-Dimethyl-3-oxa-18,18,19,19-tetradehydro-6a-carba-protaglandin-$I_2$

5. (5Z)-(16RS)-16,20-Dimethyl-3-oxa-18,18,19,19-tetradehydro-6a-carba-prostaglandin-$I_2$

6. (5E)-(16RS)-16,20-Dimethyl-3-oxa-19,19,20,20-tetradehydro-6a-carba-prostaglandin-$I_2$

7. (5Z)-(16RS)-16,20-Dimethyl-3-oxa-19,19,20,20-tetradehydro-6a-carba-prostaglandin-$I_2$

8. (5E)-(15RS)-15-Methyl-3-oxa-18,18,19,19-tetradehydro-6a-carba-prostaglandin-$I_2$

9. (5E)-3-oxa-18,18,19,19-tetradehydro-6a-carba-prostaglandin-$I_2$

10. (5E)-(16RS)-16,19-Dimethyl-3-oxa-18,19-didehydro-6a-carba-prostaglandin-$I_2$

11. (5E)-(16RS)-16-Methyl-3-oxa-18,18,19,19-tetradehydro-6a-carba-prostaglandin-$I_2$ methyl ester

12. (5E)-(16RS)-16-Methyl-3-oxa-18,18,19,19-tetradehydro-N-methansulphonyl-6a-carba-prostaglandin-$I_2$-carboxamide

13. (5E)-(16RS)-16-Methyl-3-oxa-18,18,19,19-tetradehydro-6a-carba-prostaglandin-$I_2$-acetylamide

14. (5E)-(16RS)-16-Methyl-3-oxa-18,18,19,19-tetradehydro-6a-carba-prostaglandin-$I_2$-carboxamide

15. (5E)-(16RS)-16-Methyl-3-oxa-18,18,19,19-tetradehydro-6a-carba-prostaglandin-$I_2$ trishydroxymethylaminomethane salt

16. Process for the preparation of carbacyclin derivatives of general formula I, characterised in that, in a known manner, a compound of general formula II

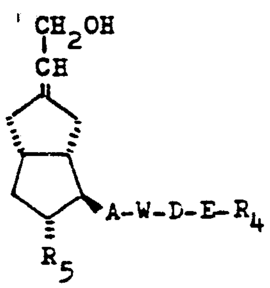

(II)

wherein $R_4$, $R_5$, A, W, D and E have the meanings given above, is etherified in the presence of a base, if necessary after protection of the free hydroxy group, with a haloacetic acid derivative of general formula III

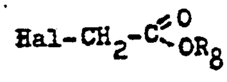

(III)

wherein Hal is chlorine or bromine and $R_8$ is alkyl of 1-4 carbon atoms or an alkali metal and, if necessary, in random sequence separated into isomers and/or treated to remove the protecting group from a hydroxy group and/or a free hydroxy group is esterified or etherified and/or a free carboxyl group is esterified and/or an esterified carboxyl group is hydrolysed or a carboxyl group is converted to an amide or to a salt with a physiologically compatible base.

17. Pharmaceutical compositions, which comprise one or several compounds according to Claim 1 together with conventional additives or carriers.

**Claims** (for the Contracting State AT)

Process for the preparation of carbacyclin derivatives of general formula I,

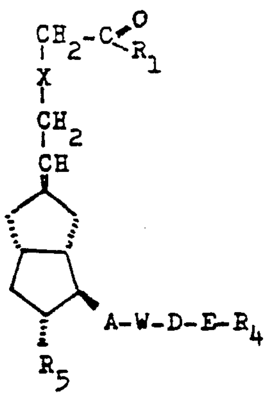

(I)

wherein

$R_1$ is the group $OR_2$, wherein $R_2$ is hydrogen, alkyl of 1-10 carbon atoms, (which can be substituted by fluorine, chlorine, bromine, phenyl, dimethylamino, diethylamino, methoxy or ethoxy), cycloalkyl of 5-6 carbon atoms, (which can be substituted by alkyl of 1-4 carbon atoms), phenyl, 2- or 3-furyl, 2- or 3-thienyl or 2- 3- or 4-pyridyl, or $R_1$ is the group $NHR_3$, where $R_3$ is a carboxylic acid or sulphonic acid group of 1-15 carbon atoms, or is hydrogen ;

X is oxygen ;

A is a $—CH_2—CH_2—$, trans-CH=CH- or —C≡C-group ;

W is a free or functionally modified hydroxymethylene group, or a free or functionally modified

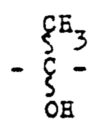

group, in which the OH-group can be in the alpha- or beta position ;

D is a straight or branched chain, saturated or unsaturated alkylene group of 1-10 carbon atoms, which can be substituted by fluorine, 1,2-methylene or 1,1-trimethylene

E is a —C≡C-group or a $—CR_6=CR_7$-group, wherein $R_6$ and $R_7$ are hydrogen or alkyl of 1-5 carbon atoms

$R_4$ is alkyl of 1-10 carbon atoms, cycloalkyl of 5-6 carbon atoms, phenyl, 2- or 3-furyl, 2- or 3-thienyl or 2-, 3- or 4-pyridyl ;

$R_5$ is a free or functionally modified hydroxy group,

and when $R_2$ is hydrogen, salts of these compounds with physiologically compatible bases, characterised in that, in a known manner, a compound of general formula II

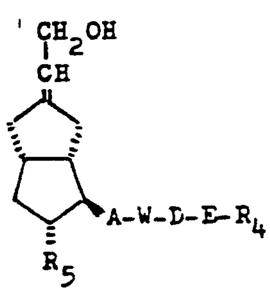

(II)

wherein $R_4$, $R_5$, A, W, D and E have the meaning given above, is etherified in the presence of a base, if necessary after protection of the free hydroxy group, with a haloacetic acid derivative of general formula III

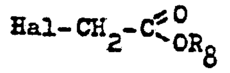

(III)

wherein Hal is chlorine or bromine and $R_8$ is alkyl of 1-4 carbon atoms or an alkali metal and, if necessary, in random sequence separated into isomers and/or treated to remove the protecting group from a hydroxy group and/or a free hydroxy group is esterified or etherified and/or a free carboxyl group is esterified and/or an esterified carboxyl group is hydrolysed or a carboxyl group is converted to an amide or to a salt with a physiologically compatible base.

**Revendications** (pour les Etats contractants : BE, CH, DE, FR, GB, IT, LI, LU, NL, SE)

1. Dérivés de la carbacycline répondant à la formule générale (I)

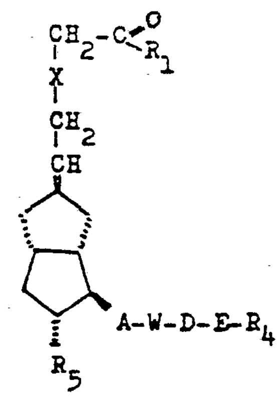

(I)

dans laquelle

$R_1$ représente un radical $—OR_2$ dans lequel $R_2$ représente l'hydrogène, un alkyle contenant de 1 à 10 atomes de carbone, éventuellement porteur d'un atome de fluor, de chlore ou de brome ou d'un radical phényle, diméthylamino, diéthylamino, méthoxy ou éthoxy, un cycloalkyle contenant 5 ou 6 atomes de carbone, éventuellement porteur d'un alkyle en $C_1$-$C_4$, un phényle, un furyle-2 ou -3, un thiényle-2 ou -3 ou un pyridyle-2, -3 ou -4, ou un radical $—NHR_3$ dans lequel $R_3$ désigne un radical d'acide carboxylique ou sulfonique contenant de 1 à 15 atomes de carbone, ou un atome d'hydrogène,

X représente l'oxygène,

A représente un radical $—CH_2—CH_2—$, un radical —CH=CH-trans ou un radical —C≡C—,

W représente un radical hydroxyméthylène libre ou modifié fonctionnellement, ou un radical

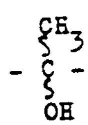

libre ou modifié fonctionnellement, le radical —OH ayant la configuration α ou la configuration β,

D représente un radical alkylène en $C_1$-$C_{10}$, saturé ou insaturé, linéaire ou ramifié, qui peut porter des atomes de fluor, un radical méthylène en 1,2 ou un radical triméthylène en 1,1,

E représente un radical —C≡C- ou un radical $—CR_6=CR_7$- dans lequel $R_6$ et $R_7$ représentent chacun un atome d'hydrogène ou un radical alkyle contenant de 1 à 5 atomes de carbone,

$R_4$ représente un alkyle contenant de 1 à 10 atomes de carbone, un cycloalkyle à 5 ou 6 atomes de carbone, un phényle, un furyle-2 ou -3, un thiényle-2 ou -3, ou un pyridyle-2, -3 ou -4, et

$R_5$ représente un groupe hydroxy libre ou modifié fonctionnellement,

et, lorsque $R_2$ désigne un atome d'hydrogène, les sels qu'ils forment avec des bases acceptables du point de vue physiologique.

2. Méthyl-16 oxa-3 tétradéhydro-18,18,19,19 carba-6a prostaglandine $I_2$ (5E)-(16RS).
3. Méthyl-16 oxa-3 tétradéhydro-18,18,19,19 carba-6a prostaglandine $I_2$ (5Z)-(16RS).
4. Diméthyl-16,20 oxa-3 tétadéhydro-18,18,19,19 carba-6a prostaglandine $I_2$ (5E)-(16RS).
5. Diméthyl-16,20 oxa-3 tétradéhydro-18,18,19,19 carba-6a prostaglandine $I_2$ (5Z)-(16RS).
6. Diméthyl-16,20 oxa-3 tétradéhydro-19,19,20,20 carba-6a prostaglandine $I_2$ (5E)-(16RS).
7. Diméthyl-16,20 oxa-3 tétradéhydro-19,19,20,20 carba-6a prostaglandine $I_2$ (5Z)-(16RS).
8. Méthyl-15 oxa-3 tétradéhydro-18,18,19,19 carba-6a prostaglandine $I_2$ (5E)-(15RS).
9. Oxa-3 tétradéhydro-18,18,19,19 carba-6a prostaglandine $I_2$ (5E).
10. Diméthyl-16,19 oxa-3 didéhydro-18,19 carba-6a prostaglandine $I_2$ (5E)-(16RS).

11. Ester méthylique de la méthyl-16 oxa-3 tétradéhydro-18,18,19,19 carba-6a prostaglandine $I_2$ (5E)-(16RS).

12. N-Méthane-sulfonyl-amide de la méthyl-16 oxa-3 tétradéhydro-18,18,19,19 carba-6a prostaglandine $I_2$ (5E)-(16RS).

13. Acétylamide de la méthyl-16 oxa-3 tétradéhydro-18,18,19,19 carba-6a prostaglandine $I_2$ (5E)-(16RS).

14. Amide de la méthyl-16 oxa-3 tétradéhydro-18,18,19,19 carba-6a prostaglandine $I_2$ (5E)-(16RS).

15. Sel d'amino-tris-(hydroxyméthyl)-méthane de la méthyl-16 oxa-3 tétrahydro-18,18,19,19 carba-6a prostaglandine $I_2$ (5E)-(16RS).

16. Procédé de préparation de dérivés de la carbacycline de formule générale (I), procédé caractérisé en ce qu'on éthérifie de manière connue, en présence d'une base, un composé répondant à la formule générale (II)

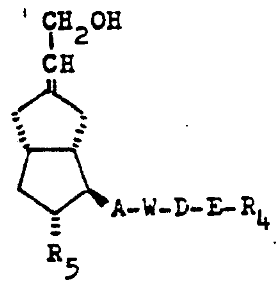

(II)

dans laquelle $R_4$, $R_5$, A, W, D, et E ont les significations indiquées ci-dessus, éventuellement après en avoir protégé d'éventuels groupes hydroxy libres, au moyen d'un dérivé d'acide halogéno-acétique répondant à la formule générale (III)

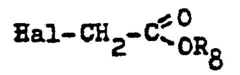

(III)

dans laquelle Hal représente un atome de chlore ou de brome et $R_8$ représente un alkyle contenant de 1 à 4 atomes de carbone ou un métal alcalin, et ensuite, le cas échéant, en opérant dans l'ordre que l'on veut, on sépare les isomères et/ou on libère des radicaux hydroxy protégés et/ou on estérifie ou éthérifie des radicaux hydroxy libres et/ou on estérifie un radical carboxy libre et/ou on saponifie un radical carboxy estérifié ou on transforme un radical carboxy en un radical carboxamide ou, au moyen d'une base acceptable du point de vue physiologique, en un sel.

17. Médicament constitué d'un ou plusieurs composés selon la revendication 1 ainsi que d'adjuvants et excipients usuels.

**Revendication** (pour l'Etat contractant AT)

Procédé de préparation de dérivés de la carbacycline de formule générale (I)

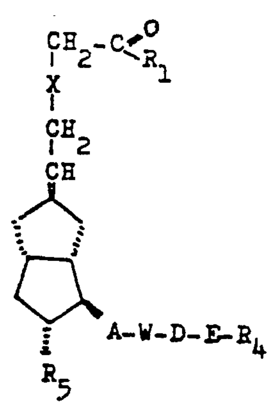

(I)

dans laquelle

$R_1$ représente un radical $—OR_2$ dans lequel $R_2$ représente l'hydrogène, un alkyle contenant de 1 à 10 atomes de carbone, éventuellement porteur d'un atome de fluor, de chlore ou de brome ou d'un radical phényl, diméthylamino, diéthylamino, méthoxy ou éthoxy, un cycloalkyle contenant 5 ou 6 atomes

de carbone, éventuellement porteur d'un alkyle en $C_1$-$C_4$, un phényle, un furyle-2 ou -3, un thiényle-2 ou -3 ou un pyridyle-2, -3 ou -4, ou un radical —$NHR_3$ dans lequel $R_3$ désigne un radical d'acide carboxylique ou sulfonique contenant de 1 à 15 atomes de carbone, ou un atome d'hydrogène,

X représente l'oxygène,

A représente un radical —$CH_2$—$CH_2$—, un radical —CH=CH-trans ou un radical —C≡C—,

W représente un radical hydroxyméthylène libre ou modifié fonctionnellement, ou un radical

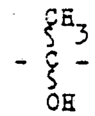 (III)

libre ou modifié fonctionnellement, le radical -OH ayant la configuration α ou la configuration β,

D représente un radical alkylène en $C_1$-$C_{10}$, saturé ou insaturé, linéaire ou ramifié, qui peut porter des atomes de fluor, un radical méthylène en 1,2 ou un radical triméthylène en 1,1,

E représente un radical —C≡C- ou un radical —$CR_6$=$CR_7$- dans lequel $R_6$ et $R_7$ représentent chacun un atome d'hydrogène ou un radical alkyle contenant de 1 à 5 atomes de carbone,

$R_4$ représente un alkyle contenant de 1 à 10 atomes de carbone, un cycloalkyle à 5 ou 6 atomes de carbone, un phényle, un furyle-2 ou -3, un thiényle-2 ou -3, ou un pyridyle-2, -3 ou -4, et

$R_5$ représente un groupe hydroxy libre ou modifié fonctionnellement,

et, lorsque $R_2$ désigne un atome d'hydrogène, les sels qu'ils forment avec des bases acceptables du point de vue physiologique, procédé caractérisé en ce qu'on éthérifie de manière connue, en présente d'une base, un composé répondant à la formule générale (II)

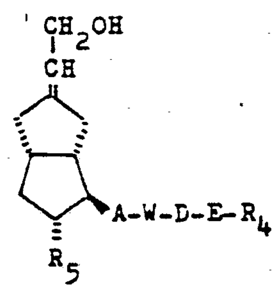

dans laquelle $R_4$, $R_5$, A, W, D et E ont les significations indiquées ci-dessus, éventuellement après en avoir protégé d'éventuels groupes hydroxy libres, au moyen d'un dérivé d'acide halogéno-acétique répondant à la formule générale (III)

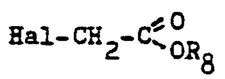

dans laquelle Hal représente un atome de chlore ou de brome et $R_\alpha$ représente un alkyle contenant de 1 à 4 atomes de carbone ou un métal alcalin, et ensuite, le cas échéant, en opérant dans l'ordre que l'on veut, on sépare les isomères et/ou on libère des radicaux hydroxy protégés et/ou on estérifie ou éthérifie des radicaux hydroxy libres et/ou on estérifie un radical carboxy libre et/ou on saponifie un radical carboxy estérifié ou on transforme un radical carboxy en un radical carboxamide ou, au moyen d'une base acceptable du point de vue physiologique, en un sel.